# FASCICULE DE BREVET EUROPEEN

(11) **EP 2 379 005 B1**
(45) Date de publication et mention de la délivrance du brevet: **04.03.2015**
(21) Numéro de dépôt: 09801493.9
(22) Date de dépôt: 03.12.2009
(51) Int. Cl.: A61F 2/00

(54) **DISPOSITIF IMPLANTABLE ET IMPLANT PROTHETIQUE COMPRENANT AU MOINS UN TEL DISPOSITIF**
IMPLANTIERBARE VORRICHTUNG UND PROTHESENIMPLANTAT MIT MINDESTENS EINER SOLCHEN VORRICHTUNG
IMPLANTABLE DEVICE AND PROSTHETIC IMPLANT INCLUDING AT LEAST ONE SUCH DEVICE

(30) Priorité: 21.01.2009 FR 0950361
(43) Date de publication de la demande: 26.10.2011
(73) Titulaire: Cousin Biotech, 59117 Wervicq Sud (FR)
(72) Inventeur: RICHARD, François, F-75116 Paris (FR); DALLE, Valery, F-59170 Croix (FR); SOLECKI, Gilles, F-59390 Lannoy (FR)
(74) Mandataire: Balesta, Pierre
(86) Numéro de dépôt international: PCT/FR2009/052398
(87) Numéro de publication internationale: WO 2010/084254

(56) Documents cités:
- WO-A-2005/122954
- US-A1- 2003 045 774

## Description

La présente invention concerne le domaine technique des dispositifs implantables comprenant un premier élément textile ayant une première et une seconde portions délimitant une longueur donnée et un élément filaire de réglage de ladite longueur solidarisé dans lesdites première et seconde portions ainsi qu'un implant prothétique, notamment pour l'incontinence urinaire, équipé d'un tel dispositif.

L'incontinence urinaire est définie comme toute perte involontaire d'urine dont se plaint le patient. L'incontinence urinaire peut être de trois types : d'effort, par impériosité ou mixte, c'est-à-dire d'effort avec une composante variable d'impériosité.

L'incontinence urinaire d'effort est caractérisée par une fuite involontaire d'urine par le méat urétral, non précédée du besoin d'uriner, qui survient à l'occasion d'un effort tel que la toux, le rire, l'éternuement, le saut, la course, le soulèvement de charges ou toute autre activité physique augmentant la pression intra-abdominale. Elle est due le plus souvent à une hyper mobilité cervico-urétrale lors de l'effort et plus rarement à une insuffisance sphinctérienne.

Parmi les principaux traitements chirurgicaux proposés pour corriger l'incontinence urinaire d'effort, on distingue le traitement par soutènement urétral à l'aide d'une bandelette. Cette bandelette est mise en place sans tension, sous la partie moyenne de l'urètre, et est destinée à supporter l'urètre pendant l'effort, empêchant ainsi toute fuite d'urine. On distingue plusieurs types de bandelettes associées à des voies chirurgicales différentes. On connaît ainsi la bandelette TVT (Tension-Free Vaginal tape) associée au traitement chirurgical par voie rétro-pubienne et la bandelette TOT (Trans-Obturator Tape) associée au traitement chirurgical par voie trans-obturatrice. L'introduction de la voie trans-obturatrice (TOT) en remplacement de la voie rétro-pubienne (TVT) a permis de réduire significativement le taux de complications vésicales, hémorragiques et digestives.

Dans le cas de la voie rétro-pubienne, une incision est pratiquée dans le vagin, sous l'urètre, et deux incisions sont pratiquées dans la peau de l'abdomen. La bandelette TVT est alors placée sous l'urètre à l'aide d'un instrument spécifique puis remonte de part et d'autre de la vessie pour venir s'ancrer dans la paroi abdominale. Cette méthode est délicate à mettre en oeuvre. De plus, il existe un risque de perforation de la vessie lors de la manipulation des aiguilles utilisées pour la pose de la bandelette. Des conséquences graves ont également été constatées telles qu'une perforation de l'artère iliaque ou encore de l'intestin grêle, entraînement des complications viscérales parfois mortelles. C'est la raison pour laquelle il est indispensable, avec cette méthode chirurgicale de pratiquer une cystoscopie.

Dans le cas de la voie trans-obturatrice, des incisions sont réalisées, d'une part, sur la paroi vaginale et, d'autre part, sur la face interne de chaque cuisse en regard des trous obturateurs. La bandelette TOT utilisée dans le cadre de cette méthode comporte une partie centrale disposée sous l'urètre et est conçue pour traverser les trous obturateurs et venir ensuite s'ancrer par ses extrémités au niveau de l'aine. Bien que cette méthode présente moins de risques de perforation d'organes importants que la méthode par voie rétro-pubienne, elle ne supprime pas complètement le risque de complications vasculaires en raison de la longueur du trajet occupé par la bandelette dans le corps.

Une dernière technique consiste à utiliser une mini bandelette destinée à être disposée sous l'urètre de manière identique au procédé par la voie trans-obturatrice, à la différence qu'une fois implantée, la mini bandelette ne franchit ni l'espace rétro-pubien, ni le foramen obturateur, ni la membrane obturatrice.

Les bandelettes du type TOT ou TVT comportent des projections latérales et longilignes, de l'ordre de 10 cm à 15 cm, se projetant de la partie centrale de soutènement de l'urètre et destinées à venir s'accrocher en des points distants de ladite partie centrale, de sorte que les frottements exercés sur les trajets anatomiques maintiennent la bandelette ancrée dans sa position d'implantation.

Les dispositifs représentés dans WO 2005 122 954 concernent des écharpes de support à longueur/tension réglable.

La mini bandelette ne comporte pas de telles projections longilignes suivant des trajets anatomiques éloignés de la partie centrale, de sorte que des moyens d'ancrage implantables sont disposés sur celle-ci.

On connaît ainsi dans EP 1.324.705 B1 des mini bandelettes munies à chacune de leurs extrémités d'une ancre de laquelle se projettent de multiples saillies arrangées dans une configuration en arbre de Noël. Lesdites saillies fléchissent dans le sens d'introduction de l'ancre afin de faciliter le passage de celle-ci dans les tissus mous, en particulier dans le tissu mou fibro-graisseux de la zone rétro-pubienne. L'ancrage est assuré par la résistance opposée par les saillies déployées lorsqu'une traction est exercée sur l'ancre dans un sens opposé à celui de son introduction. Ces ancres étant injectées sont rigides et donc blessantes pour les tissus. Il n'est pas possible de les retirer sans disséquer les tissus une fois que les ailettes se sont déployées et sont en appui plan sur une couche de tissu, tel que représenté à la figure 8C.

Lors de l'implantation, quelle que soit la technique utilisée et décrite précédemment, la patiente étant sous anesthésie locale, le praticien dispose dans un premier temps la bandelette dans sa position d'implantation, la partie centrale étant notamment sous l'urètre, et demande à la patiente de tousser, si il observe des fuites urinaires, il doit repositionner la bandelette et donc facilement retrouver les extrémités de celles-ci. Or, une fois implantée, seule une petite portion de la partie centrale de la bandelette est visible pour le praticien. Les moyens d'ancrage connus dans l'état de la technique, et notamment l'ancre de type arbre de Noël décrite ci-dessus, ne comprennent pas de moyens de guidage permettant au praticien de retrouver facilement et de manière sécurisée les extrémités de la bandelette pour ajuster la position de cette dernière. Le chirurgien peut avoir également besoin d'ajuster la longueur de la bandelette soutenant l'urètre afin de la soutenir davantage sans modifier l'emplacement des moyens d'ancrage déjà implantés disposés aux extrémités de la bandelette.

La présente invention a pour objet selon un premier aspect un dispositif implantable résolvant tout ou partie des problèmes précités.

Le dispositif implantable selon l'invention comprend un premier élément textile ayant une première et une seconde portions délimitant une longueur donnée et un élément filaire de réglage de ladite longueur solidarisé dans lesdites première et seconde portions. De manière caractéristique, le premier élément textile est tubulaire, notamment une tresse, et l'élément filaire forme une première boucle entourant extérieurement le premier élément tubulaire dans la première portion, et en passant à l'intérieur du premier élément tubulaire, une seconde boucle entourant extérieurement le premier élément tubulaire dans la seconde portion, en sorte qu'une traction exercée sur au moins l'une des extrémités libres dudit élément filaire permet le rapprochement desdites première et seconde portions et corrélativement la réduction de la longueur séparant lesdites première et seconde portions du premier élément textile.

Ce dispositif d'ajustement de la longueur entre deux portions du premier élément tubulaire est simple et peu onéreux à fabriquer. De plus, il est simple à mettre en oeuvre par le praticien. Une fois la longueur séparant lesdites portions ajustée, la longueur excédentaire d'élément filaire à l'extérieur du premier élément tubulaire est coupée par le praticien. L'élément filaire, à l'exception des tronçons formant les boucles extérieures, de préférence plaquées contre le premier élément tubulaire, est disposé à l'intérieur du premier élément tubulaire, en sorte qu'il ne peut être accroché ou ne risque pas de gêner les tissus ou organes environnants.

L'élément filaire étant solidarisé dans les première et seconde portions, quelle que soit sa position : desserré ou serré, il est apte à coulisser dans lesdites portions à travers les jours du premier élément tubulaire textile et à l'intérieur de ce dernier.

L'élément filaire présente une longueur très largement supérieure à sa largeur. L'élément filaire peut être un mono-filament, un fil multi-filamentaire ou une tresse de faible diamètre. L'élément filaire peut être dans un matériau partiellement ou totalement résorbable, absorbable ou dans un matériau non résorbable ou non absorbable. L'élément filaire est de préférence à base d'un ou plusieurs polymères choisis parmi les polymères suivants : polypropylène, polyéthylène-téréphtalate, polyéthylène, polyamide 4-6 ou 6-6, PLLA de forme D ou L (polyacide lactique de forme D ou L), copolymère d'acide lactique et glycolique, copolymère d'acide lactique et glycolique en proportion respective 80/20 (PLGA 80/20), copolymère d'acide lactique et glycolique en proportion respective 90/10 (PLGA 90/10), copolymère d'acide lactique de forme L et de forme D, copolymère d'acide lactique de forme L et de forme D en proportion respective 80/20 (PLDA 80/20), copolymère d'acide lactique de forme L et de forme D en proportion respective 90/10 (PLDA 90/10), copolymère de différents Poly(alpha-hydroxy esters).

L'élément filaire présente un diamètre externe inférieur à la taille des jours ou ouvertures délimitées par les fils formant le premier élément tubulaire. De préférence, l'élément filaire a un diamètre extérieur de l'ordre de 0,05 mm à 3,0 mm.

L'élément filaire est disposé à l'intérieur du premier élément tubulaire, de préférence à l'aide d'un tube inséré à travers l'un des jours dudit premier élément. Le diamètre interne dudit tube étant choisi en sorte de recevoir au moins deux brins dudit élément filaire à la fois.

Avantageusement, le premier élément étant tubulaire, le rapprochement de deux portions séparant celui-ci créent une corolle facilitant l'ancrage mécanique dans les tissus environnants et améliorant le blocage desdites portions dans leur position rapprochée. De préférence, la formation d'une corolle d'ancrage régulière est obtenue lorsque le premier élément tubulaire est une tresse.

En effet, les fils formant la tresse ayant déjà une configuration en hélice au repos, leur mise en rotation lors de la formation de la corolle est facilitée.

Le blocage desdites portions dans leur position rapprochée est obtenu du fait des frottements engendrés entre l'élément filaire et les jours du premier élément tubulaire. Ce blocage peut être renforcé à l'aide d'un noeud effectué à l'aide d'au moins une extrémité libre dudit élément filaire.

Le premier élément tubulaire textile peut être tissé, tricoté ou tressé, et comporte des jours délimités par les fils le constituant. Le tricotage peut s'effectuer sur un métier circulaire. Ledit premier élément peut également être formés à partir d'un panneau tricoté ou tissé dont les bords sont soudés, par exemple par ultrasons, ou cousus en sorte de former un tube.
Le premier élément tubulaire comprend des fils multi-filamentaires et/ou des mono-filaments, de préférence des mono-filaments ayant un diamètre compris dans l'intervalle [0,05 ; 0,50] mm. Les fils multi-filamentaires et/ou les mono-filaments sont de préférence à base d'un ou plusieurs polymères choisis parmi les polymères suivants : polypropylène, polyéthylène, polyéthylène-téréphtalate, polyamide 6-6 ou 4-6 PLLA de forme D ou L (polyacide lactique de forme D ou L), copolymère d'acide lactique et glycolique, copolymère d'acide lactique et glycolique en proportion respective 80/20 (PLGA 80/20), copolymère d'acide lactique et glycolique en proportion respective 90/10 (PLGA 90/10), copolymère d'acide lactique de forme L et de forme D, copolymère d'acide lactique de forme L et de forme D en proportion respective 80/20 (PLDA 80/20), copolymère d'acide lactique de forme L et de forme D en proportion respective 90/10 (PLDA 90/10), copolymère de différents Poly(alpha-hydroxy esters).

De préférence, le premier élément tubulaire est une tresse. En effet, le demandeur s'est aperçu que les éléments tubulaires tressés offrent dans le cadre du dispositif selon la présente invention une meilleure stabilité dimensionnelle, une bonne mémoire de forme tout en permettant une fabrication économique et simple.

Dans une variante, l'élément filaire forme une première boucle autour de ladite première portion du premier élément textile dont les extrémités libres se prolongent à l'intérieur dudit premier élément tubulaire et sortent à l'extérieur de celui-ci au niveau de ladite seconde portion, se partagent de part et d'autre du premier élément tubulaire pour former une seconde boucle autour de ladite seconde portion, à l'opposé de ladite première boucle, et se prolongent de nouveau à l'intérieur dudit premier élément tubulaire, pour sortir à l'extérieur du premier élément tubulaire, de préférence en périphérie de ladite première boucle.

L'actionnement d'au moins une extrémité libre de l'élément filaire dans une direction opposée à celle de la seconde boucle, tout en maintenant sensiblement le premier élément tubulaire en périphérie de ladite seconde portion, sans bloquer la seconde boucle, permet le rapprochement de la première boucle vers la seconde boucle et corrélativement la réduction de la longueur séparant lesdites portions.

Dans une variante, une première extrémité libre de l'élément filaire passe sous au moins un tronçon de la première boucle, de préférence sous au moins deux tronçons de la première boucle, en sorte qu'une fois la traction exercée sur la seconde extrémité libre de l'élément filaire pour rapprocher les première et seconde portions du premier élément textile, il suffit d'exercer une traction sur ladite première extrémité libre pour bloquer la longueur réduite du premier élément tubulaire.

Dans une variante, la première extrémité du premier élément tubulaire est solidarisée ou solidarisable à un implant, notamment pour le traitement de l'incontinence urinaire, et une collerette externe d'ancrage est formée à partir d'au moins une couche d'au moins un second élément textile tubulaire enveloppant la seconde extrémité dudit premier élément tubulaire, une partie distale semi-rigide reliant les parties extrêmes du premier élément tubulaire et de la collerette.

Le second élément textile tubulaire peut présenter la même structure et la même composition que le premier élément textile tubulaire.

Les premier et second éléments tubulaires ainsi que la collerette externe d'ancrage font office de moyens d'ancrage et de guidage implantables simples à utiliser, peu onéreux, faciles à fabriquer, et susceptibles d'être posés et maintenus dans leur position d'implantation sans tension. De plus, ces moyens d'ancrage et de guidage sont moins traumatisants pour les tissus mous que les dispositifs d'ancrage connus et plus faciles à repositionner.

Avantageusement, les moyens d'ancrage et de guidage étant dans un matériau textile, ils favorisent la fibrose, laquelle renforce l'ancrage mécanique dudit implant dans sa position d'implantation. De plus, lesdits moyens d'ancrage étant par définition souples, ils sont moins traumatisants pour les tissus dans lesquels ils sont introduits que des dispositifs d'ancrage rigides.

La collerette textile souple fait ici office d'ancre mécanique. Le premier élément tubulaire se prolonge à partir de la partie distale semi-rigide à l'intérieur de la collerette.

La collerette étant formée à partir d'un second élément tubulaire est creuse ; elle peut donc servir avantageusement de moyen de positionnement en recevant l'extrémité d'un outil chirurgical, notamment d'un ancillaire. Le praticien positionne ladite collerette externe d'ancrage, solidarisée à une seconde extrémité du premier élément textile, en glissant l'extrémité d'un ancillaire à l'intérieur du premier élément jusqu'à ce que ce dernier vienne en butée avec l'extrémité distale de ladite collerette externe d'ancrage.

La collerette a de préférence une forme générale rectangulaire au repos. Lors de son introduction dans les tissus mous, elle a tendance à se déformer, permettant une insertion moins traumatique dans les tissus qu'une ancre rigide. Lorsqu'on exerce une traction sur la collerette dans une direction opposée à son sens d'introduction, elle a alors tendance à se comprimer et à raccourcir pour adopter une configuration sensiblement trapézoïdale, la base de la collerette à l'opposé de la partie distale vient alors en appui sur les tissus environnants.

Plus la collerette a tendance à se comprimer et adopter une forme trapézoïdale, plus la résistance au déplacement de la collerette dans les tissus dans une direction opposée au sens de son introduction, est élevée. Le demandeur a ainsi observé que cet effet est amélioré lorsque la collerette est formée à partir d'une tresse. En effet, lorsque l'on pince une tresse au repos en deux zones distincts puis que l'on rapproche ces deux zones, la tresse se comprime facilement puis s'aplatie pour former un disque.

Lors de l'implantation desdits moyens d'ancrage et de guidage dans le tissu mou fibro-graisseux rétro-pubien, la collerette textile, par définition poreuse, emmagasine dans son volume intérieur de la graisse, ce qui améliore sa résistance au déplacement, notamment dans une direction opposée au sens de son introduction et donc corrélativement sa capacité d'ancrage.

De préférence, ces moyens d'ancrage et de guidage sont adaptés pour l'ancrage et le guidage d'implants dans des tissus mous.

En fonctionnement, une fois la collerette externe d'ancrage implantée, le praticien peut rapprocher la première portion de la seconde portion simplement en exerçant une traction sur l'une des extrémités libres de l'élément filaire dans une direction opposée au sens d'introduction de ladite collerette externe d'ancrage. Il n'est pas nécessaire de maintenir en position le premier élément tubulaire à l'aide d'un outil de placement, tel qu'un ancillaire, lorsqu'une traction est exercée sur au moins l'une des extrémités libres de l'élément filaire. La force de traction nécessaire pour rapprocher les première et seconde portions est inférieure à la force de traction nécessaire pour déplacer la collerette externe d'ancrage dans des tissus mous.

De préférence, les premier et second éléments textiles tubulaires étant constitués totalement ou partiellement de fils thermo-fusibles, la partie distale est formée par thermofusion des parties extrêmes superposées desdits éléments tubulaires.

Dans une variante, la collerette externe d'ancrage comporte de deux à quatre couches superposées.

Le nombre de couches superposées a un impact, d'une part, sur la rigidité de la collerette, et donc sur sa capacité à s'ancrer mécaniquement en particulier dans des tissus mous et, d'autre part, sur la stabilité dimensionnelle de la collerette.

Dans une variante, tous les éléments textiles tubulaires sont identiques.

Les premier et second éléments tubulaires sont formés à partir d'un seul élément textile tubulaire, de préférence une tresse.

Au moins certains des fils constitutifs du ou des éléments textiles tubulaires de la collerette ont un titrage supérieur à celui des fils constitutifs du premier élément tubulaire en sorte d'améliorer la rigidité de celle-ci et sa capacité d'ancrage mécanique dans les tissus, en particulier dans les tissus mous. La collerette textile conserve cependant une excellente souplesse lui permettant de se déformer et de ne pas déchirer les tissus lors de l'insertion du dispositif dans le corps.

Dans une variante, deux couches adjacentes de la collerette constituent un repli d'un même élément tubulaire.

Dans une variante, le premier élément tubulaire comporte vers sa première extrémité une ouverture apte à permettre le passage d'un outil de placement dans ledit premier élément tubulaire jusqu'à venir en butée intérieurement contre la partie distale.

Dans une variante, le premier élément tubulaire est une tresse et l'ouverture apte à permettre le passage d'un outil de placement est formée par l'un des jours délimités par les fils constituant ladite tresse.

Dans ce cas, la tresse est de préférence fabriquée en sorte de présenter des jours déformables ; l'outil chirurgical est en effet inséré dans un jour en repoussant les fils adjacents les uns contre les autres. Des outils chirurgicaux variés et standards peuvent ainsi être utilisés.

Le premier élément tubulaire fait office de gaine recevant l'outil chirurgical et assure ainsi la fonction de moyen de guidage. Le praticien remonte alors l'outil de placement à l'intérieur dudit premier élément tubulaire vers la collerette jusqu'à venir en butée intérieure contre la partie distale. Lors d'une intervention chirurgicale, le praticien peut ainsi facilement repérer l'emplacement de la collerette implantée et ajuster de manière sécurisée la position d'implantation de l'implant prothétique, en particulier s'agissant d'un implant prothétique pour le traitement de l'incontinence urinaire.

Dans une variante, la partie distale a une forme annulaire apte à recevoir en butée l'extrémité de l'organe de placement, notamment l'extrémité pointue d'un ancillaire.

Cette disposition permet de renforcer le serrage de l'extrémité de l'outil de placement et donc de faciliter la manipulation et le positionnement de la collerette.

La présente invention a pour objet, selon un deuxième aspect, un implant prothétique, notamment textile, comprenant au moins un dispositif implantable selon l'une des variantes de réalisation précitées.

Dans une variante, l'implant prothétique comprend une bandelette implantable, notamment pour l'incontinence urinaire, dont les deux extrémités sont équipées dudit dispositif implantable.

Dans une variante, la bandelette implantable est réalisée dans le premier élément textile tubulaire.

Dans une variante, la bandelette implantable comporte une partie centrale sensiblement plane obtenue du fait de l'introduction dans le premier élément tubulaire d'une pièce interne, notamment textile, qui est sensiblement plane et qui est apte à déformer localement la configuration tubulaire dudit premier élément.

De préférence, la pièce interne est fixée au premier élément tubulaire.

Dans une variante, la bandelette implantable comporte une partie centrale sensiblement plane obtenue du fait de la fixation entre elles des deux parois résultant de la déformation en largeur et de l'aplatissement du premier élément tubulaire.

Les deux précédentes variantes décrivent des moyens de mise à plat de la partie centrale tubulaire de la bandelette, notamment pour le cas où la partie centrale est destinée à être disposée sous l'urètre lorsque la bandelette est destinée à la cure de l'incontinence urinaire.

Ces moyens de mise à plat évitent l'effet « fil à couper le beurre » de la bandelette lorsque celle-ci est tendue dans sa position d'implantation. De préférence, la bandelette n'est pas élastique.

La présente invention sera mieux comprise à la lecture de deux exemples de réalisation cités à titre non limitatif, et illustrés dans les figures ci-après, annexées à la présente, et dans lesquelles :
- la figure 1 est une vue de dessus d'un implant prothétique comprenant à chacune de ses extrémités un premier exemple de dispositif selon la présente invention au repos ;
- la figure 2 est une vue de face de la collerette représentée à la figure 1 ;
- la figure 3 est une vue de dessus d'un implant prothétique comprenant à chacune de ses extrémités un premier exemple de dispositif représenté à la figure 2 et en fonctionnement ;
- la figure 4 est une vue de dessus d'un implant prothétique comprenant à chacune de ses extrémités un second exemple de dispositif selon la présente invention au repos.

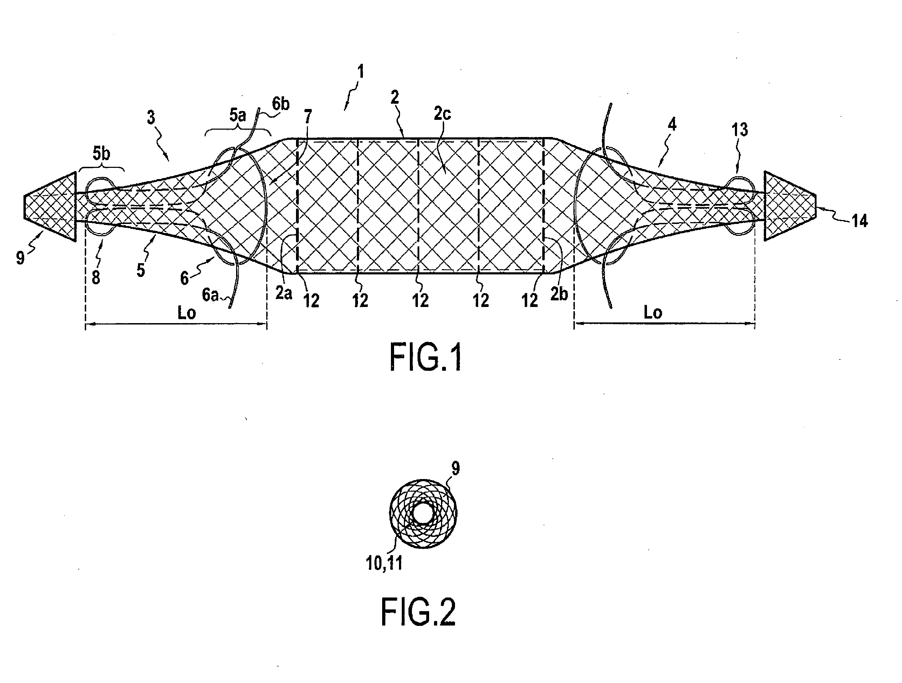

L'implant prothétique 1 représenté à la figure 1 comprend une bandelette implantable 2, notamment pour l'incontinence urinaire, dont les deux extrémités 2a,2b comprennent un dispositif implantable 3,4 selon la présente invention. Le second dispositif 4 étant dans cet exemple précis identique au premier dispositif 3, pour des raisons de simplification, seul le premier dispositif 3 disposé à la première extrémité 2a de la bandelette 2 est décrit. Le premier dispositif 3 comprend un premier élément textile tubulaire 5 ayant une première portion 5a et une seconde portion 5b délimitant une longueur donnée Lo au repos et un premier élément filaire 6 de réglage de ladite longueur Lo solidarisé dans lesdites portions 5a,5b. Ledit premier élément filaire 6 forme une première boucle 7 autour de la première portion 5a du premier élément tubulaire 5 dont les extrémités libres 6a,6b se prolongent à l'intérieur dudit premier élément tubulaire 5 et sortent à l'extérieur de celui-ci au niveau de la seconde portion 5b, se partagent de part et d'autre du premier élément tubulaire 5 pour former une seconde boucle 8 autour de la seconde portion 5b, à l'opposé de la première boucle 7, et se prolongent à nouveau à l'intérieur dudit premier élément tubulaire 5, pour sortir à l'extérieur du premier élément 5, dans cet exemple précis en périphérie de la première boucle 7 dans la première portion 5a. De préférence, le premier élément tubulaire 5 est une tresse. Le premier dispositif 3 comprend également une collerette externe d'ancrage 9 formée à partir d'au moins une couche du premier élément tubulaire 5 replié sur lui-même, les parties extrêmes 10 du premier élément 5 replié sur lui-même sont solidarisées en sorte de former une partie distale 11 semi-rigide. La collerette externe 9 d'ancrage est dans cet exemple formée de deux couches superposées du premier élément tubulaire 5. De préférence, le premier élément tubulaire 5 comprend en totalité ou en partie des mono-filaments dans un ou plusieurs polymères fusibles en sorte de pouvoir fondre partiellement ou totalement les parties extrêmes 10 du premier élément replié sur lui-même et former ladite partie distale 11. De préférence, les mono-filaments sont en polypropylène (PP) et ont un diamètre de l'ordre de 0,15 mm (tresse 48 fuseaux avec un pas de 50 mm). Le premier élément 5 étant un textile de forme tubulaire, il comprend des ouvertures ou jours de l'ordre du millimètre déformables et une partie distale annulaire 11 permettant le passage d'un outil de placement, notamment l'extrémité pointue d'un ancillaire, dans le premier élément 5 jusqu'à venir en butée intérieurement contre la partie distale 11. La bandelette implantable 2 est réalisée dans le premier élément tubulaire 5 et comporte une partie centrale 2c sensiblement plane obtenue du fait de l'introduction dans le premier élément 5 d'une pièce interne textile (non représentée), de préférence un tricot, qui est sensiblement plane et apte à déformer localement la configuration tubulaire du premier élément 5. Cette configuration sensiblement plane est renforcée grâce à la fixation entre elles des deux parois dudit premier élément 5, selon les lignes de solidarisation transversales 12 résultant de la déformation en largeur et de l'aplatissement du premier élément tubulaire 5. Le second dispositif 4 disposé à la seconde extrémité 2b de la bandelette 2 est également formé à partir du premier élément tubulaire 5 de la même façon que le premier dispositif 3. Le second élément filaire 13 est agencé de la même façon dans le second dispositif 4 que le premier élément filaire 6 dans le premier dispositif 3.

En fonctionnement, lors d'une implantation, le praticien insert à travers un jour du premier dispositif 3 et à l'intérieur du premier élément tubulaire 5, un outil de placement pourvue d'une extrémité pointue jusqu'à venir en butée intérieurement contre la partie distale 11 de la première collerette externe d'ancrage 9. Le praticien peut ainsi disposer cette première collerette d'ancrage 9 dans les tissus mous fibro-graisseux de la zone rétro-pubienne. Lors du déplacement de la collerette 9 dans son sens d'introduction, les tissus mous environnants compriment les parties latérales de la première collerette 9 puisque celle-ci est avantageusement déformable facilitant ainsi son déplacement dans le sens d'introduction en minimisant les déchirures de tissus sur son passage. La même opération est reproduite pour le placement de la seconde collerette externe d'ancrage 14 du second dispositif 4 dans les tissus mous de la zone rétro-pubienne. Si le praticien souhaite repositionner les collerettes externes d'ancrage 9,14 afin de les remonter davantage et améliorer le soutènement de l'urètre, destiné à reposer sur la bandelette 2, il est possible de réintroduire l'organe de placement à travers le premier élément tubulaire 5 et d'ajuster leur position. Bien que le premier élément textile 5 soit tubulaire et gaine l'outil de placement afin d'éviter d'accrocher des structures anatomiques environnantes, le praticien peut ajuster la longueur séparant les première 5a et seconde 5b portions en actionnant l'une ou l'autre des deux extrémités libres 6a,6b du premier élément filaire 6 du premier dispositif 3. Le praticien peut répéter cette opération, si nécessaire, en actionnant le second élément filaire 13 de sorte de rapprocher les première et seconde portions du second dispositif 4.

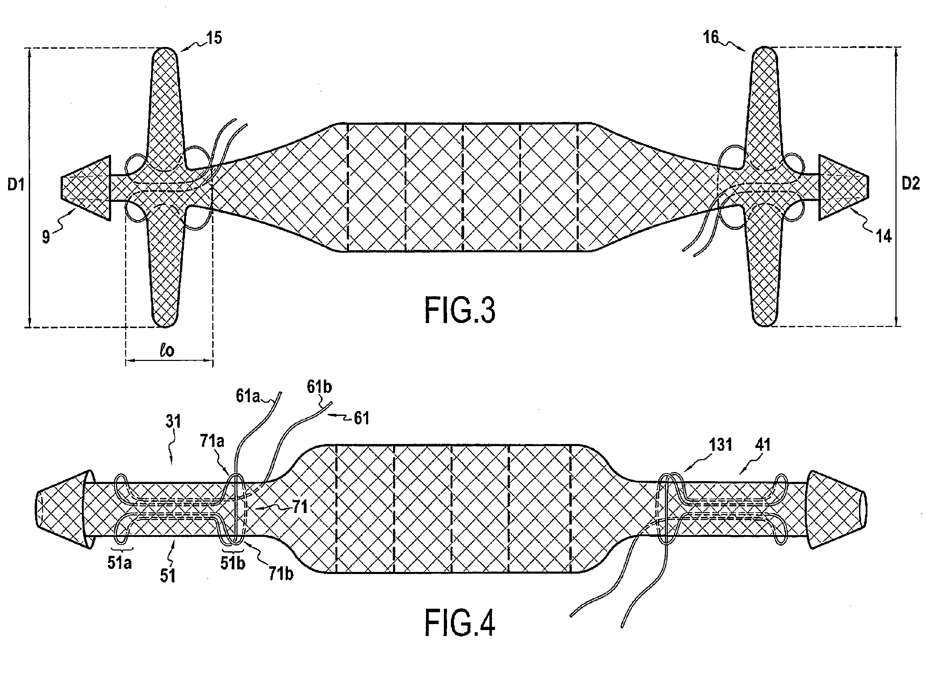

La figure 3 représente les premier 3 et second 4 dispositifs en fonctionnement, les longueurs séparant les premières 5a et seconde 5b portions ayant été réduites à ℓo. Le rapprochement des premières 5a et secondes 5b portions dans les premier 3 et second 4 dispositifs permet la formation de deux corolles externes d'ancrage 15,16 renforçant l'ancrage mécanique des collerettes externes d'ancrage 9,14 disposées aux extrémités des premier 3 et second 4 dispositifs. A la figure 3, les corolles externes d'ancrage 15,16 sont représentées avec un diamètre externe maximal D₁,D₂ sensiblement identique puisque la longueur Lo séparant les première 5a et seconde 5b portions sur le premier dispositif 3 est sensiblement également à celle séparant les première et seconde portions du second dispositif 4. Les corolles externes d'ancrage 15,16 peuvent présenter des diamètres externes inférieurs à ceux représentés à la figure 3 selon la traction exercée par le praticien et l'ajustement du rapprochement des premières et secondes portions. Il est également possible de prévoir des longueurs séparant les première et seconde portions différentes entre le premier 3 et le second 4 dispositif en sorte de faire varier encore davantage le diamètre externe D₁,D₂ des corolles d'ancrage 15,16.

Les éléments filaires 6,13 effectuant plusieurs aller-retour entre les premières 5a et secondes 5b portions, les forces de frottement engendrées permettent de bloquer les corolles externes d'ancrage 15,16, de sorte que le praticien peut couper les extrémités libres 6a,6b excédentaires des premier 6 et second 13 éléments filaires se projetant à l'extérieur du premier élément tubulaire 5.

Néanmoins, dans une variante illustrée à la figure 4, il est possible de bloquer le premier élément filaire 61 en passant la première extrémité libre 61a de ce dernier sous le tronçon 71b et le tronçon 71a de la première boucle 71, en sorte qu'une fois la traction exercée sur la seconde extrémité libre 61b du premier élément filaire 61 pour rapprocher les première 51a et seconde 51b portions du premier élément 51 dans le premier dispositif 31, il suffit d'exercer une traction sur la première extrémité libre 61a pour bloquer la longueur réduite du premier élément 51. La même opération peut être effectuée à partir du second élément filaire 131 disposé dans le second dispositif 4.

## Revendications

1. Dispositif implantable (3,4,31,41) comprenant un premier élément textile (5,51) ayant une première (5a,51a) et une seconde (5b,51b) portions délimitant une longueur donnée (Lo) et un élément filaire (6,13,61,131) de réglage de ladite longueur (Lo) solidarisé dans lesdites première et seconde portions **caractérisé en ce que** le premier élément textile (5,51) est tubulaire, notamment une tresse, et **en ce que** l'élément filaire (6,13,61,131) forme une première boucle (7,71) entourant extérieurement le premier élément tubulaire (5,51) dans ladite première portion (5a,51a), et en passant à l'intérieur de celui-ci, une seconde boucle (8) entourant extérieurement le premier élément tubulaire (5,51) dans ladite seconde portion (5b,51b), en sorte qu'une traction exercée sur au moins l'une des extrémités libres (6a,6b) dudit élément filaire permet le rapprochement desdites première (5a,51a) et seconde (5b,51b) portions et corrélativement la réduction de la longueur séparant lesdites première et seconde portions du premier élément textile (5,51).

2. Dispositif implantable (3,4,31,41) selon la revendication 1, **caractérisé en ce que** l'élément filaire (6,13,61,131) forme une première boucle (7,71) autour de ladite première portion (5a,51a) du premier élément textile (5,51) dont les extrémités libres se prolongent à l'intérieur dudit premier élément tubulaire (5,51) et sortent à l'extérieur de celui-ci au niveau de ladite seconde portion (5b,51b), se partagent de part et d'autre du premier élément tubulaire (5,51) pour former une seconde boucle (8) autour de ladite seconde portion (5b,51b), à l'opposé de ladite première boucle (7,71), et se prolongent de nouveau à l'intérieur dudit premier élément tubulaire (5,51), pour sortir à l'extérieur dudit premier élément tubulaire (5,51), de préférence en périphérie de ladite première boucle (7,71).

3. Dispositif implantable (31,41) selon l'une ou l'autre des revendications 1 et 2, **caractérisé en ce qu'**une première extrémité libre (61a) de l'élément filaire (61) passe sous au moins un tronçon (71b) de la première boucle (71), de préférence sous au moins deux tronçons (71a,71b) de la première boucle (71), en sorte qu'une fois la traction exercée sur la seconde extrémité libre (61b) de l'élément filaire (61) pour rapprocher les première (51a) et seconde (51b) portions du premier élément textile (51), il suffit d'exercer une traction sur ladite première extrémité libre (61a) pour bloquer la longueur réduite du premier élément tubulaire (51).

4. Dispositif implantable (3,4,31,41) selon l'une des revendications 1 à 3, **caractérisé en ce que** la première extrémité du premier élément tubulaire (5,51) est solidarisée ou solidarisable à un implant (1), notamment pour le traitement de l'incontinence urinaire, et **en ce qu'**une collerette externe d'ancrage (9,14) est formée à partir d'au moins une couche d'au moins un second élément textile tubulaire enveloppant la seconde extrémité dudit premier élément tubulaire, une partie distale semi-rigide (11) reliant les parties extrêmes (10) du premier élément tubulaire (5,51) et de la collerette (9,14).

5. Dispositif implantable (3,4,31,41) selon la revendication 4, **caractérisé en ce que** la collerette externe d'ancrage (9,14) comporte de deux à quatre couches superposées.

6. Dispositif implantable (3,4,31,41) selon l'une ou l'autre des revendications 4 et 5, **caractérisé en ce que** tous les éléments textiles tubulaires (5,51) sont identiques.

7. Dispositif implantable (3,4,31,41) selon l'une des revendications 4 à 6, **caractérisé en ce que** deux couches adjacentes de la collerette (9,14) constituent un repli d'un même élément tubulaire(5,51).

8. Dispositif implantable (3,4,31,41) selon l'une des revendications 4 à 7 **caractérisé en ce que** le premier élément tubulaire (5,51) comporte vers sa première extrémité une ouverture apte à permettre le passage d'un outil de placement dans ledit premier élément tubulaire (5,51) jusqu'à venir en butée intérieurement contre la partie distale (11).

9. Dispositif implantable (3,4,31,41) selon l'une des revendications 4 à 8, **caractérisé en ce que** la partie distale (11) a une forme annulaire apte à recevoir en butée l'extrémité de l'organe de placement, notamment l'extrémité pointue d'un ancillaire.

10. Implant prothétique (1), notamment textile, comprenant au moins un dispositif implantable (3,4,31,41) selon l'une des revendications 1 à 9.

11. Implant prothétique (1) selon la revendication 10, **caractérisé en ce qu'**il comprend une bandelette implantable (2), notamment pour l'incontinence urinaire, dont les deux extrémités (2a,2b) sont équipées dudit dispositif implantable (3,4,31,41).

12. Implant prothétique (1) selon la revendication 11, **caractérisé en ce que** la bandelette implantable (2) est réalisée dans le premier élément textile tubulaire (5,51).

13. Implant prothétique (1) selon la revendication 12, **caractérisé en ce que** la bandelette implantable (2) comporte une partie centrale (2c) sensiblement plane obtenue du fait de l'introduction dans le premier élément tubulaire (5,51) d'une pièce interne, notamment textile, qui est sensiblement plane et qui est apte à déformer localement la configuration tubulaire dudit premier élément (5,51).

14. Implant prothétique (1) selon la revendication 13, **caractérisé en ce que** la bandelette implantable (2) comporte une partie centrale (2c) sensiblement plane obtenue du fait de la fixation entre elles des deux parois résultant de la déformation en largeur et de l'aplatissement du premier élément tubulaire (5,51).

## Patentansprüche

1. Implantierbare Vorrichtung (3, 4, 31, 41), umfassend ein erstes Textilelement (5, 51), das einen ersten (5a, 51 a) und einen zweiten (5b, 51 b) Abschnitt aufweist, die eine gegebene Länge (Lo) abgrenzen, und ein fadenförmiges Element (6, 13, 61, 131) zum Einstellen der Länge (Lo), das in dem ersten und zweiten Abschnitt befestigt ist, **dadurch gekennzeichnet, dass** das erste Textilelement (5, 51) röhrenförmig ist, insbesondere ein Geflecht, und dass das fadenförmige Element (6,13, 61,131) eine erste Schlaufe (7, 71), die das erste röhrenförmige Element (5, 51) in dem ersten Abschnitt (5a, 51 a) außen umgibt und im Inneren davon durchgeht, eine zweite Schlaufe (8), die das erste röhrenförmige Element (5, 51) in dem zweiten Abschnitt (5b, 51 b) außen umgibt, bildet, so dass ein Zug, der an mindestens einem der freien Enden (6a, 6b) des fadenförmigen Elements ausgeübt wird, das Annähern des ersten (5a, 51 a) und des zweiten (5b, 51 b) Abschnitts und dementsprechend das Reduzieren der Länge ermöglicht, die den ersten und den zweiten Abschnitt des ersten Textilelements (5, 51) trennt.

2. Implantierbare Vorrichtung (3, 4, 31, 41) nach Anspruch 1, **dadurch gekennzeichnet, dass** das fadenförmige Element (6, 13, 61, 131) eine erste Schlaufe (7, 71) um den ersten Abschnitt (5a, 51 a) des ersten Textilelements (5, 51) bildet, deren freien Enden sich im Inneren des ersten röhrenförmigen Elements (5, 51) erstrecken und aus diesem an dem zweiten Abschnitt (5b, 51 b) heraustreten, indem sie sich an beiden Seiten des ersten röhrenförmigen Elements (5, 51) trennen, um eine zweite Schlaufe (8) um den zweiten Abschnitt (5b, 51 b), die der ersten Schlaufe (7,71) gegenüberliegt, zu bilden und sich wieder in dem ersten röhrenförmigen Element (5, 51) erstrecken, um aus dem ersten röhrenförmigen Element (5, 51), vorzugsweise am Rand der ersten Schlaufe (7, 71), herauszutreten.

3. Implantierbare Vorrichtung (31, 41) nach einem der Ansprüche 1 und 2, **dadurch gekennzeichnet, dass** ein erstes freies Ende (61 a) des fadenförmigen Elements (61) unter mindestens einem Abschnitt (71 b) der ersten Schlaufe (71), vorzugsweise unter mindestens zwei Abschnitten (71 a, 71 b) der ersten Schlaufe (71) derart verläuft, dass es, sobald der Zug auf das zweite freie Ende (61 b) des fadenförmigen Elements (61) ausgeübt wird, um den ersten (51 a) und den zweiten (51 b) Abschnitt des ersten Textilelements (51) näher zu bringen, ausreicht, einen Zug auf das erste freie Ende (61 a) auszuüben, um die reduzierte Länge des ersten röhrenförmigen Elements (51) zu blockieren.

4. Implantierbare Vorrichtung (3, 4, 31, 41) nach einem der Ansprüche 1 bis 3, **dadurch gekennzeichnet, dass** das erste Ende des ersten röhrenförmigen Elements (5, 51) an einem Implantat (1), insbesondere zur Behandlung der Harninkontinenz, befestigt ist oder befestigt werden kann, und dass ein äußerer Verankerungskragen (9, 14) aus mindestens einer Lage mindestens eines zweiten röhrenförmigen Textilelements gebildet ist, das das zweite Ende des ersten röhrenförmigen Elements umhüllt, wobei ein halbstarrer distaler Abschnitt (11) die Endabschnitte (10) des ersten röhrenförmigen Elements (5, 51) und des Kragens (9, 14) verbindet.

5. Implantierbare Vorrichtung (3, 4, 31, 41) nach Anspruch 4, **dadurch gekennzeichnet, dass** der äußere Verankerungskragen (9, 14) zwei bis vier übereinander angeordnete Lagen aufweist.

6. Implantierbare Vorrichtung (3, 4, 31, 41) nach einem der Ansprüche 4 und 5, **dadurch gekennzeichnet, dass** alle röhrenförmigen Textilelemente (5, 51) identisch sind.

7. Implantierbare Vorrichtung (3, 4, 31, 41) nach einem der Ansprüche 4 bis 6, **dadurch gekennzeichnet, dass** zwei benachbarte Lagen des Kragens (9, 14) einen Umschlag eines gleichen röhrenförmigen Elements (5, 51) bilden.

8. Implantierbare Vorrichtung (3, 4, 31, 41) nach einem der Ansprüche 4 bis 7, **dadurch gekennzeichnet, dass** das erste röhrenförmige Element (5, 51) in Richtung seines ersten Endes eine Öffnung aufweist, die geeignet ist, das Durchführen eines Plazierungswerkzeugs in dem ersten röhrenförmigen Element (5, 51), bis dieses innen an dem distalen Teil (11) in Anschlag gelangt, zu ermöglichen.

9. Implantierbare Vorrichtung (3, 4, 31, 41) nach einem der Ansprüche 4 bis 8, **dadurch gekennzeichnet, dass** der distale Abschnitt (11) eine Ringform aufweist, die geeignet ist, das Ende des Plazierungsorgans, insbesondere das spitze Ende eines Hilfsinstruments anschlagend aufzunehmen.

10. Prothetisches Implantat (1), insbesondere Textilimplantat, umfassend mindestens eine implantierbare Vorrichtung (3, 4, 31, 41) nach einem der Ansprüche 1 bis 9.

11. Prothetisches Implantat (1) nach Anspruch 10, **dadurch gekennzeichnet, dass** es ein implantierbares Band (2), insbesondere für die Harninkontinenz aufweist, dessen beide Enden (2a, 2b) mit der implantierbaren Vorrichtung (3, 4, 31, 41) ausgestattet sind.

12. Prothetisches Implantat (1) nach Anspruch 11, **dadurch gekennzeichnet, dass** das implantierbare Band (2) aus dem ersten röhrenförmigen Textilelement (5, 51) gebildet ist.

13. Prothetisches Implantat (1) nach Anspruch 12, **dadurch gekennzeichnet, dass** das implantierbare Band (2) einen im Wesentlichen ebenen mittleren Teil (2c) aufweist, der dadurch erhalten wird, dass ein Innenteil, insbesondere textiles Innenteil, das im Wesentlichen eben ist und das geeignet ist, die röhrenförmige Gestalt des ersten Elements (5, 51) lokal zu verformen, in das erste röhrenförmige Element (5, 51) eingeführt wird.

14. Prothetisches Implantat (1) nach Anspruch 13, **dadurch gekennzeichnet, dass** das implantierbare Band (2) einen im Wesentlichen ebenen mittleren Teil (2c) aufweist, der dadurch erhalten wird, dass die beiden Wände, die sich aus der Breitenverformung und aus der Abflachung des ersten röhrenförmigen Elements (5, 51) ergeben, miteinander befestigt werden.

## Claims

1. An implantable device (3, 4, 31, 41) comprising a first textile element (5, 51) having first and second portions (5a, 51a; 5b, 51b) defining a given length (Lo) and a filamentary element (6, 13, 61, 131) for adjusting said length (Lo) secured in said first and second portions, the device being **characterized in that** the first textile element (5, 51) is tubular, in particular a braid, and **in that** the filamentary element (6, 13, 61, 131) forms a first loop (7, 71) externally surrounding the first tubular element (5, 51) in said first portion (5a, 51a) and, by passing inside the tubular element, a second loop (8) externally surrounding the first tubular element (5, 51) in said second portion (5b, 51b), such that traction exerted on at least one of the free ends (6a, 6b) of said filamentary element enables said first and second portions (5a, 51a; 5b, 51b) to be moved towards each other and correspondingly enables the length between said first and second portions of the first textile element (5, 51) to be shortened.

2. An implantable device (3, 4, 31, 41) according to claim 1, **characterized in that** the filamentary element (6, 13, 61, 131) forms a first loop (7, 71) around said first portion (5a, 51a) of the first textile element (5, 51) having its free ends extending inside said first tubular element (5, 51) and extends out therefrom at said second portion (5b, 51b), lying on either side of the first tubular element (5, 51) to form a second loop (8) around said second portion (5b, 51b) opposite from said first loop (7, 71), and extending once more inside said first tubular element (5, 51) to extend out from said first tubular element (5, 51), preferably at the periphery of said first loop (7, 71).

3. An implantable device (31, 41) according to claim 1 or claim 2, **characterized in that** a first free end (61a) of the filamentary element (61) passes under at least one segment (71b) of the first loop (71), preferably under at least two segments (71a, 71b) of the first loop (71), such that when traction is exerted on the second free end (61b) of the loop element (61) in order to move the first and second portions (51a, 51b) of the first textile element (51) towards each other, it suffices to exert traction on said first free end (61a) in order to block the shortened length of the first tubular element (51).

4. An implantable device (3, 4, 31, 41) according to any one of claims 1 to 3, **characterized in that** the first end of the first tubular element (5, 51) is secured or securable to an implant (1), in particular for treating urinary incontinence, and **in that** an outer anchor collar (9, 14) is formed from at least one layer of at least one second tubular textile element surrounding the second end of said first tubular element, a semirigid distal portion (11) connecting together the extreme portions (10) of the first tubular element (5, 51) and of the collar (9, 14).

5. An implantable device (3, 4, 31, 41) according to claim 4, **characterized in that** the outer anchor collar (9, 14) comprises two to four superposed layers.

6. An implantable device (3, 4, 31, 41) according to claim 4 or claim 5, **characterized in that** all of the tubular textile elements (5, 51) are identical.

7. An implantable device (3, 4, 31, 41) according to any one of claims 4 to 6, **characterized in that** two adjacent layers of the collar (9, 14) constitute a fold in a single tubular element (5, 51).

8. An implantable device (3, 4, 31, 41) according to any one of claims 4 to 7, **characterized in that** the first tubular element (5, 51) includes an opening towards its first end, which opening is suitable for passing a positioning tool in said first tubular element (5, 51) until it comes into abutment internally against the distal portion (11).

9. An implantable device (3, 4, 31, 41) according to any one of claims 4 to 8, **characterized in that** the distal portion (11) has an annular shape suitable for receiving the end of the positioning member in abutment, in particular the pointed end of an ancillary instrument.

10. A prosthetic implant (1), in particular a textile implant, comprising at least one implantable device (3, 4, 31, 41) according to any one of claims 1 to 9.

11. A prosthetic implant (1) according to claim 10, **characterized in that** it comprises an implantable strip (2), in particular for urinary incontinence, both ends (2a, 2b) of the strip being fitted with said implantable device (3, 4, 31, 41).

12. A prosthetic implant (1) according to claim 11, **characterized in that** the implantable strip (2) is made in the first tubular textile element (5, 51).

13. A prosthetic implant (1) according to claim 12, **characterized in that** the implantable strip (2) includes a substantially plane central portion (2c) obtained by inserting an internal part into the first tubular element (5, 51), in particular a textile part, which internal part is substantially plane and is suitable for locally deforming the tubular configuration of said first element (5, 51).

14. A prosthetic implant (1) according to claim 13, **characterized in that** the implantable strip (2) includes a substantially plane central portion (2c) obtained by fastening together the two walls that result from deforming the first tubular element (5, 51) widthwise and flattening it.
